# EUROPEAN PATENT APPLICATION

(11) **EP 0 856 328 A2**
(43) Date of publication of application: **05.08.1998**
(21) Application number: 98300074.6
(22) Date of filing: 07.01.1998
(51) Int. Cl.: A61M 16/10

(54) **Fluid-treatment assemblies**

(30) Priority: 31.01.1997 GB 9702058
(71) Applicant: Smiths Industries Public Limited Company, London, NW11 8DS (GB)
(72) Inventor: Pagan, Eric, Hythe, Kent, CT21 6DA (GB)
(74) Representative: Flint, Jonathan McNeill

(57) **Abstract**

A medical breathing filter has a flexible tubular outer housing (5) having one port (2) connected to the patient and another port (6) connected to receive ventilation gas. A flexible, tubular filter (10) is mounted coaxially in the housing (5) and has one end sealed with the housing between the two ports. The opposite end (15) of the filter (10) is closed so that gas flowing through the housing flows through the thickness of the filter.

## Description

This invention relates to fluid-treatment assemblies of the kind comprising an outer housing and a fluid treatment element located in the housing between a first port and a second port.

Filters are used in medical breathing applications, to filter air or gas supplied to and from a patient. In order to ensure that bacteria and viruses are completely blocked by the filter, it must have a high efficiency, which leads to an increased flow resistance. Flow resistance can be minimized by increasing the area of the filter, so medical filters with acceptable filtering efficiency and flow resistance tend to be bulky. This is generally undesirable because it obstructs access to the patient and is more likely to impede other equipment in the vicinity of the patient. Heat and moisture exchangers (HMEs) also tend to be bulky when they have a low flow resistance and a high exchange efficiency.

It is an object of the present invention to provide an improved fluid-treatment assembly.

According to the present invention there is provided a :fluid treatment assembly of the above-specified kind, characterised in that the housing is of tubular shape, that the fluid treatment element has a tubular portion extending coaxially within the housing, that one end of the tubular portion is sealed with the housing between the first and second ports, and that the other end of the tubular portion is closed such that fluid passing through the housing between the first and second ports must flow through the thickness of the fluid treatment element.

The outer housing and the tubular portion may be flexible. The tubular portion may include an outer tubular portion extending from the one end and an inner tubular portion joined with the other end of the outer tubular portion and extending coaxially within the outer tubular portion. The outer tubular portion preferably tapers from the one end to the other end, the inner tubular portion tapering in the opposite direction such as to provide a tapering annular recess between the outer and inner tubular portions. The tubular portion may be corrugated. Alternatively, the tubular portion may be a radially-pleated ruff of conical shape. Alternatively, the tubular portion may be provided by a plurality of conical elements joined with one another end-to-end. Alternatively, the tubular portion may be provided by a helically-wound strip of material. Alternatively, the tubular portion may be provided by a tube of filter material that is creased through its thickness to increase its surface area and flexibility. The fluid treatment element is preferably a medical breathing gas filter and may include an HME element, such as a foam.

Filter assemblies according to the present invention will now be described, by way of example, with reference to the accompanying drawings, in which:
- Figure 1: is a side elevation view of a filter assembly in use;
- Figure 2: is a perspective view of the filter assembly of Figure 1;
- Figure 3: is a cross-sectional side elevation view of an alternative filter assembly;
- Figure 4: is a perspective view of a filter element for another assembly;
- Figures 5 and 6: show steps in the manufacture of the filter element of Figure 4;
- Figure 7: is a perspective view of a further filter element, ;
- Figure 8: is a cross-sectional side elevation view of a modified form of the filter element shown in Figure 7;
- Figure 9: is a cross-sectional side elevation view of another alternative filter assembly ;
- Figure 10: is a perspective view of a further alternative filter element;
- Figure 11: is a perspective view showing a step in the manufacture of the filter element of Figure 10; and
- Figure 12: is a cross-sectional side elevation of another alternative filter assembly.

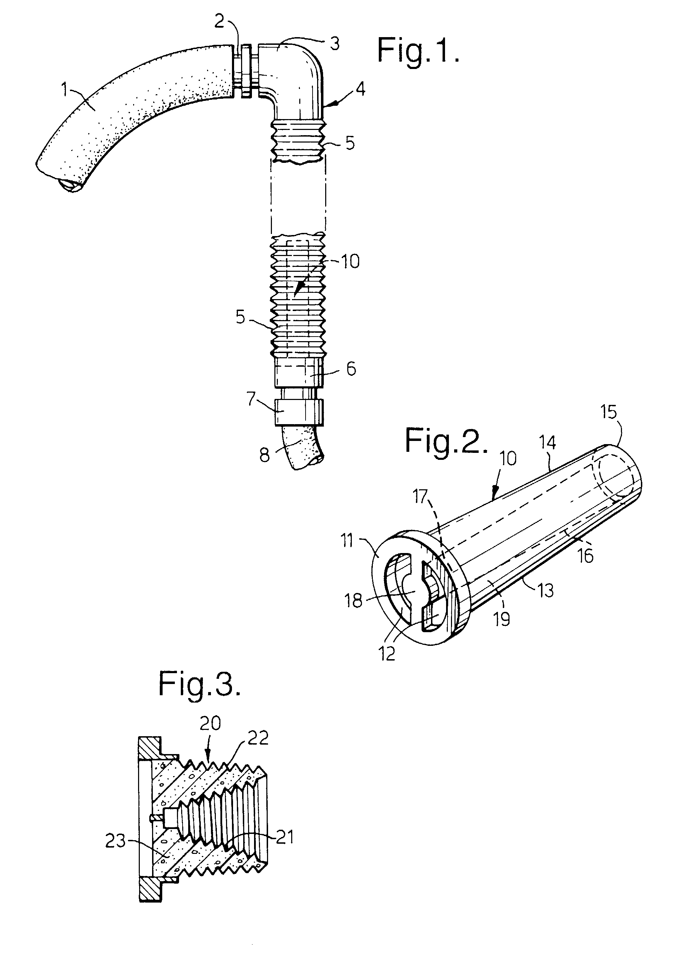

With reference first to Figures 1 and 2, there is shown an assembly including a tracheal tube 1 (only a part of which is shown) having a port or connector 2 at its machine end, which is connected to a right-angle coupling 3 at the patient end of a catheter mount 4. The catheter mount has a length of flexible, corrugated tubing 5 extending from the patient end connector 2 to a machine end port or connector 6, which, in turn, is connected to a connector 7 at the patient end of ventilation tubing 8 by which medical breathing gas is supplied to and from the assembly.

The catheter mount 4 includes a filter assembly 10 extending longitudinally within the catheter mount forwardly along about half its length. The catheter mount 4 provides an outer housing for the filter assembly 10 and the connectors 2 and 6 form ports for gas passage through the filter assembly. The filter assembly 10 has a rigid, circular mounting plate 11 at its rear end, which is perforated by two D-shape apertures 12. A filter element 13 of a conventional filter paper or fabric material is joined at one end to the forward side of the plate 11 and is closed at its other end. The filter element 13 comprises an outer tube 14 of conical shape, tapering slightly to a reduced diameter at its other, forward end 15 where it is joined with an inner tube 16. The term "cone" or "conical" is used to include part-conical, such as frusto-conical shapes. The inner tube 16 is similarly tapered and extends rearwardly coaxially within the outer tube 14, having a reduced diameter at its rear end 17. The inside of the inner tube 16 is open at the forward end of the filter element 13. The filter element 13, in this example, is formed as a single conical member, the smaller end being everted within the larger end to form the two concentric outer and inner tubes 14 and 16. Alternatively, the inner and outer tubes could be formed separately and subsequently joined together. The rear end 17 of the inner tube 16 is sealed closed, such as by being joined with a central boss 18 on the plate 11. The two apertures 12 open into the annular recess 19 between the outer and inner tubes 14 and 16. The filter mounting plate 11 is sealed around its outer edge into the machine end coupling 6 so that all gas flowing along the catheter mount 4 must pass through the apertures 12 and hence through the thickness of the wall of either the outer or inner tube 14 or 16. Gas flowing to the patient flows from the annular space 19, either inwardly through the inner tube 16, or outwardly through the outer tube 14. Gas flowing in the opposite direction, from the patient, flows through the wall of the outer or inner tube 14 or 16 into the annular space 19. The external diameter of the outer tube 14 is chosen to leave sufficient clearance for gas to flow between the outside of the tube and the inside of the corrugated tubing 5. The material forming the filter element 13 is flexible or bendable, thereby enabling the catheter mount 4 to be flexed without damage either to the tubing 5 or to the filter assembly 10. The coaxial arrangement of the outer and inner filter tubes 14 and 16 enables a relatively large filter area to be achieved with a relatively small cross-sectional dimension, thereby enabling the filter to be provided within the catheter mount itself.

The assembly could also be given heat and moisture exchange properties by including an HME material, such as a flexible hygroscopic foam, in the tubing 5 forwardly of the filter element 13.

With reference now to Figure 3, there is shown a modified filter element 20 having inner and outer conical tubes 21 and 22, in which the two tubes are corrugated concentrically. This enables the same surface area to be produced in a shorter length and also improves the flexibility of the filter element. The space between inner and outer tubes 21 and 22 contains an HME material 23, such as a hygroscopic foam. This filter element 20 is located in a housing between inlet and outlet ports.

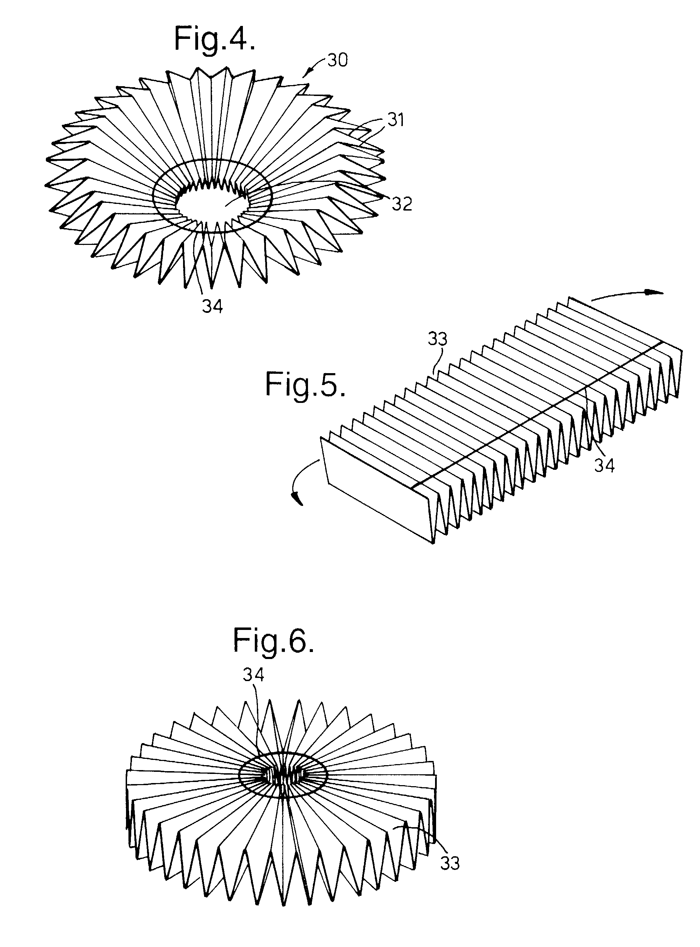

In Figure 4 there is shown an alternative tubular filter element 30 of conical shape, which has radial pleats 31 extending out from the centre 32, which is sealed closed. The outer edge of the filter element 30 is sealed to a housing between two ports (not shown), such as by means of a hot-melt adhesive. Again, this construction achieves a relatively large filter area with a relatively small cross section. The filter element 30 is made by taking a rectangular, laterally-pleated pack 33 and depositing a line 34 of a hot-melt adhesive, or similar material, longitudinally close to one edge, as shown in Figure 5. Opposite ends of the pleated pack 33 are then folded around the edge along which the line 34 of adhesive has been deposited and are sealed together to form a flat ruff, as shown in Figure 6. The centre of the ruff is then displaced down relative to the outer edge, to form the cone shape in Figure 4, and the centre is closed to prevent the direct passage of gas through it. It could be closed with additional filter material.

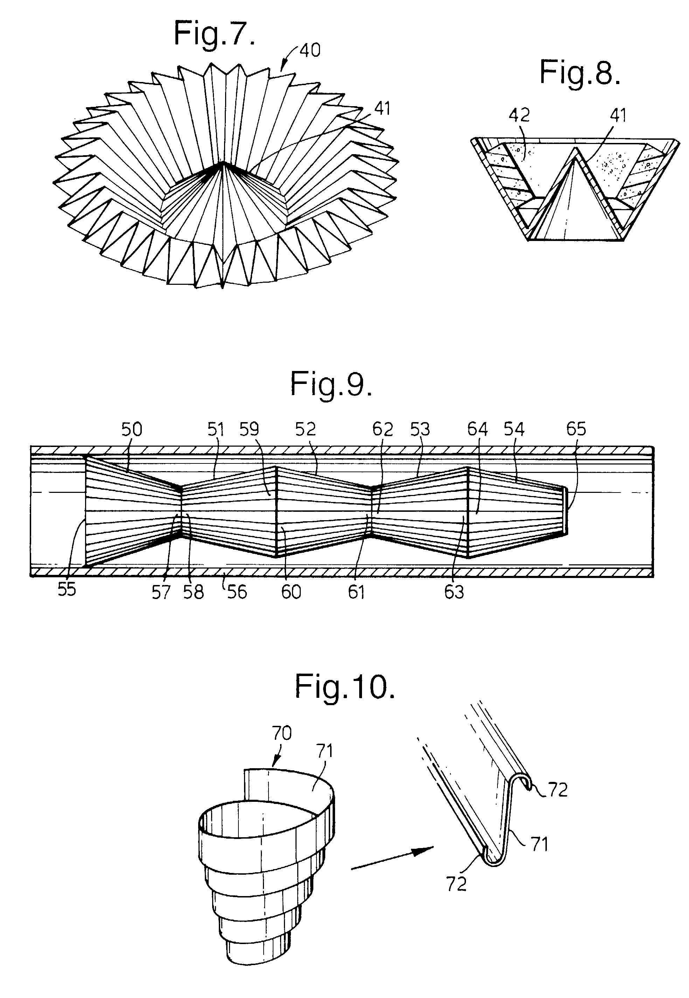

With reference now to Figure 7, there is shown a filter element 40, which is a modified form of the radially-pleated filter element 30 in Figure 4. The filter element 40 differs from the other element in that its centre 41 is inverted relative to its outside, in a similar manner to the filters shown in Figures 2 and 3. An assembly including a filter element 40 of the kind shown in Figure 7 may also include an HME element 42, as shown in Figure 8. The HME element 42 is of conical shape and is nested within the filter element.

With reference now to Figure 9, there is shown a filter assembly with five conical, radially-pleated filter elements 50 to 54 of the kind similar to that shown in Figure 4. Four of the filter elements 50 to 53 have their centres left open and are joined end-to-end. The first filter element 50 in the sequence has its outer edge 55 sealed to the inside of a housing 56, opposite ends of which provide respective inlet or outlet ports of the filter assembly. The first filter element 50 has its centre aperture 57 sealed to the centre aperture 58 of the next element 51, which is oppositely oriented to the first element 50. The outer edge 59 of the second element 51 is sealed with the outer edge 60 of the third element 52, which is oriented in the same sense as the first element 50. The centre 61 of the third element 52 is sealed with the centre 62 of the fourth element 53, which is oriented in the same sense as the second filter element 51. The outer edge 63 of the fourth filter element 53 is sealed with the outer edge 64 of the last element. The last element 54 is oriented in the same sense as the first element and has its centre 65 closed. The second to fifth filter elements 51 to 54 are elongated slightly compared with the first element 50, so that their diameters, at their larger ends are less than the internal diameter of the housing 56. When gas flow is from left to right, gas flows along the middle of the combined filter structure and flows outwardly through the wall of any of the filter elements 50 to 54 into the space between the outside of the filter elements and the housing 56. Gas passing along the housing 56 in the opposite direction flows through the filter elements 54 to 50 in the opposite sense.

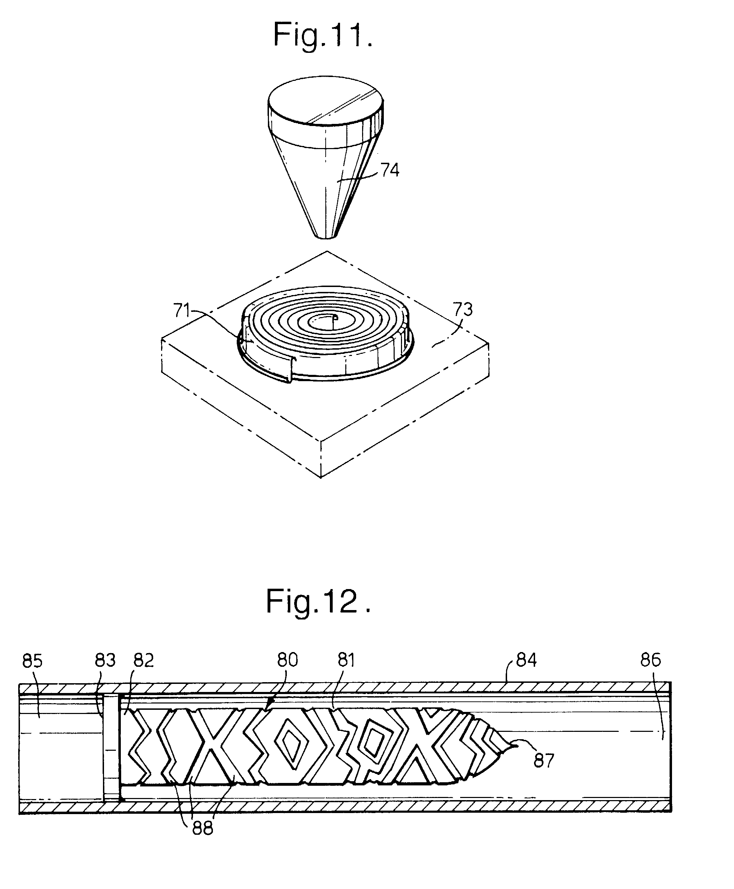

Another alternative filter element 70 is shown in Figure 10. This is filter element 70 is of conical shape and comprises a strip of filter material 71 wound helically with the width of the strip extending parallel with the axis of the helix and with adjacent turns of the strip overlapping and sealed with one another. The strip 71 may be flat or it may have channel-shape lips 72 extending along opposite edges. The filter element 70 is made in the manner shown in Figure 11, by winding the strip of filter material initially into flat coil and sealing the outer end of the strip to the underlying turn with a hot-melt adhesive. The coil is placed in a jig 73 and a conical former 74 is pushed down on the centre of the coil so as to push out the centre of the coil and form it into a conical shape. Where the strip 71 has lips 72, interlocking of the lips of adjacent turns will limit the extent of extension of the coil. A line of hot-melt adhesive, or similar material is then applied along the edge of the strip 71 where it overlaps the underlying turn of the strip, so as to seal the strip and retain it in its helical configuration. The inner, centre of the coil is also closed in the same way.

With reference now to Figure 12, there is shown a filter element 80 comprising a single tube 81 of filter material attached around its mouth at one end 82 to a perforated plate 83, which is, in turn sealed around its outside to the inside of a housing 84. The housing 84 is of tubular shape, the tube 81 extending axially along the housing and the seal at one end of the filter tube being located between ports 85 and 86 at opposite ends of the housing. The other end 87 of the filter tube 81 is closed, such as by flattening the tube and sealing the flattened sides together with an adhesive. The wall of the tube 81 is formed along its entire length with a pattern of creases 88 through the thickness of the wall, so that an indentation in the outer surface appears as a projection on the inner surface. These creases make the tube 81 more flexible and enable it to be used within a flexible outer housing, such as a catheter mount. The creases also increase the surface area of the tube compared with a tube of the same length and diameter without creases.

The filter elements described above may be replaced with HME elements of the same shape, such as of a hygroscopic paper, or by combined filter and HME elements.

## Claims

1. Fluid treatment assembly comprising an outer housing (5, 56, 84) and a fluid treatment element (10, 20, 30, 40, 50 to 54, 70, 80) located in the housing between a first port (2, 86) and a second port (6, 85), characterised in that the housing (5, 56, 84) is of tubular shape, that the fluid treatment element (10, 20, 30, 40, 50, 70, 80) has a tubular portion (14, 22, 50 to 54, 70, 81) extending coaxially within the housing, that one end (55, 82) of the tubular portion is sealed with the housing (5, 56, 84) between the first and second ports, and that the other end (15, 65, 87) of the tubular portion is closed such that fluid passing through the housing between the first and second ports must flow through the thickness of the fluid treatment element.

2. Fluid treatment assembly according to Claim 1, characterised in that the outer housing (5, 56, 84) and the tubular portion (14, 22, 50 to 54, 70, 81) are flexible.

3. Fluid treatment assembly according to Claim 1 or 2, characterised in that the tubular portion includes an outer tubular portion (14, 22) extending from the one end, and an inner tubular portion (16, 21, 41) joined with the other end of the outer tubular portion and extending coaxially within the outer tubular portion.

4. Fluid treatment assembly according to Claim 3, characterised in that the outer tubular portion (14, 22) tapers from the one end to the other end, and that the inner tubular portion (16, 21, 41) tapers in the opposite direction such as to provide a tapering annular recess (19) between the outer and inner tubular portions.

5. Fluid treatment assembly according to any one of the preceding claims, characterised in that the tubular portion (20, 30, 40, 50 to 54) is corrugated.

6. Fluid treatment assembly according to any one of Claims 1 to 3, characterised in that the tubular portion is a radially-pleated ruff (30, 40, 50 to 54) of conical shape.

7. Fluid treatment assembly according to Claim 1 or 2, characterised in that the tubular portion is provided by a plurality of conical elements (50 to 54) joined with one another end-to-end.

8. Fluid treatment assembly according to Claim 1 or 2, characterised in that the tubular portion (70) is provided by a helically-wound strip of material (71).

9. Fluid treatment assembly according to Claim 1 or 2, characterised in that the tubular portion (80) is provided by a tube (81) of filter material that is creased through its thickness to increase its surface area and flexibility.

10. Fluid treatment assembly according to any one of the preceding claims, characterised in that the fluid treatment element (10, 20, 30, 40, 50 to 54, 70, 80) is a medical breathing gas filter.

11. Fluid treatment assembly according to any one of the preceding claims, characterised in that the fluid treatment element (20, 40) includes an HME element (23, 42).

12. Fluid treatment assembly according to Claim 11 characterised in that the HME element (23, 42) is a foam.
